# EUROPEAN PATENT SPECIFICATION

(11) **EP 3 867 354 B1**
(45) Date of publication and mention of the grant of the patent: **18.03.2026**
(21) Application number: 19874445.0
(22) Date of filing: 16.10.2019
(51) Int. Cl.: C12N 5/00, A61K 35/00, C12N 5/07, C12N 5/071, C12N 5/09, G01N 33/50

(54) **COMPOSITIONS FOR AND METHODS OF PRODUCING TUMOR ORGANOIDS**
ZUSAMMENSETZUNGEN UND VERFAHREN ZUR HERSTELLUNG VON TUMORORGANOIDEN
COMPOSITIONS ET PROCÉDÉS DE PRODUCTION D'ORGANOÏDES TUMORAUX

(30) Priority: 16.10.2018 US 201862746296 P
(43) Date of publication of application: 25.08.2021
(73) Proprietor: Board of Regents, The University of Texas System, Austin, TX 78701 (US)
(72) Inventor: KALLURI, Raghu, Houston, TX 77030 (US); LEBLEU, Valerie, Houston, TX 77030 (US); CHEN, Yang, Houston, TX 77030 (US)
(74) Representative: SONN Patentanwälte GmbH & Co KG
(86) International application number: PCT/US2019/056577
(87) International publication number: WO 2020/081715

(56) References cited:
- WO-A1-2016/183183
- US-A1- 2014 336 282
- US-A1- 2017 342 385
- MAKAREEVA ELENA ET AL: "Carcinomas Contain a Matrix Metalloproteinase?Resistant Isoform of Type I Collagen Exerting Selective Support to Invasion", CANCER RESEARCH, vol. 70, no. 11, 1 June 2010 (2010-06-01), US, pages 4366 - 4374, XP055776445, ISSN: 0008-5472, DOI: 10.1158/0008-5472.CAN-09-4057
- ST�PHANIE PERRET ET AL: "Prolyl hydroxylation of collagen type I is required for efficient binding to integrin [alpha]1[beta]1 and platelet glycoprotein VI but not to [alpha]2[beta]1", JOURNAL OF BIOLOGICAL CHEMISTRY, AMERICAN SOCIETY FOR BIOCHEMISTRY AND MOLECULAR BIOLOGY, US, vol. 278, no. 32, 8 August 2003 (2003-08-08), pages 29873 - 29879, XP002626606, ISSN: 0021-9258, [retrieved on 20030527], DOI: 10.1074/JBC.M304073200
- CAREY SHAWN P. ET AL: "Three-dimensional collagen matrix induces a mechanosensitive invasive epithelial phenotype", vol. 7, no. 1, 10 February 2017 (2017-02-10), XP055930391, Retrieved from the Internet <URL:https://www.nature.com/articles/srep42088.pdf> DOI: 10.1038/srep42088
- E. LEIKINA ET AL: "Type I collagen is thermally unstable at body temperature", PROCEEDINGS OF THE NATIONAL ACADEMY OF SCIENCES, vol. 99, no. 3, 5 February 2002 (2002-02-05), pages 1314 - 1318, XP055456558, ISSN: 0027-8424, DOI: 10.1073/pnas.032307099
- HAN ET AL.: "Molecular Mechanism of Type I Collagen Homotrimer Resistance to Mammalian Collagenases", THE JOURNAL OF BIOLOGICAL CHEMISTRY, vol. 285, no. 29, 12 May 2010 (2010-05-12) - 16 July 2010 (2010-07-16), pages 22276 - 22281, XP055702651

## Description

### BACKGROUND

### 1. Field

The invention relates to methods of producing organoids as well as organoids produced thereby.

### 2. Description of Related Art

Commonly, patient tumor cells are cultured as two dimensional (2D) monolayers, which fail to recapitulate the complex composition of the tumors themselves. In addition, cells cultured as monolayers lack the interactions with stromal cells and extracellular matrix (ECM) found *in vivo.* Organoid (3D) culture allows tumor cells to proliferate, even with very low amounts of starting material. Organoids have already been instrumental in identifying potential cancer drivers and novel therapeutic targets (Boj *et al.,* 2015). However, improved and more efficient methods of generating organoids are needed.

A study of Makareeva et al. (Cancer research 70.11 (2010): 4366-4374) shows that invasive cancer cells produce homotrimeric type I collagen, which is resistant to degradation by matrix metalloproteinases (MMPs).
A further study by Perret et al. (Journal of Biological Chemistry 278.32 (2003): 29873-29879) demonstrates that prolyl hydroxylation of collagen is essential for effective binding to certain receptors, notably integrin α1β1 and platelet glycoprotein VI.
Carey et al. (Scientific reports 7.1 (2017): 42088) describe that exposure of epithelial cells to stromal collagen induces an invasive phenotype through matrix-mediated signaling and increased stiffness, requiring MT1-MMP expression and activation of Src, PI3K, and Rac1 pathways.
US 2014/0336282 A1 describes assays for identifying molecular signatures of invasive cancer subpopulations.
WO 2016/183183 A1 describes tumor organoids in collagen I gels that are used for screening agents for inhibiting cancer growth and viability.

A study of Leikina et al. (Proceedings of the National Academy of Sciences 99.3 (2002): 1314-1318) shows that human lung collagen monomers begin to unfold near body temperature and only refold below 30°C, challenging the assumption that the triple helix is stable under physiological conditions.

### SUMMARY

Described are basal cell culture media for animal or human cells, the media comprising exogenous collagen I homotrimers. In some aspects, the media are serum free. In some aspects, the media comprise a serum replacement. In some aspects, the media are supplemented with serum.

Described are cell culture additives for animal or human cells, the additives comprising exogenous collagen I homotrimers. In some aspects, the additive is an extracellular matrix, a hydrogel, a biological scaffold, or a synthetic scaffold. In some aspects, the extracellular matrix comprises laminin, entactin, and collagen IV. In some aspects, the extracellular matrix is Matrigel. In some aspects, the extracellular matrix is synthetic.

Described are cell culture surfaces for animal or human cells, the surfaces comprising exogenous collagen I homotrimers. In some aspects, the surface is a cell culture plate, a channel, a duct, a valve, or a microfluidic device. The surface may be any material used in the culture of 3D structures or tissue engineering. The surface may be any surface that can be coated with collagen homotrimers in order to support cell viability.

In one embodiment, provided herein are methods for obtaining and/or culturing an organoid, the method comprising culturing pancreatic ductal adenocarcinoma cells with an extracellular matrix in the presence of a medium, additive, or surface comprising exogenous collagen I homotrimers. In some aspects, the cells are primary cells. In some aspects, the primary cells are cancerous cells. In some aspects, the cancerous cells are pancreatic ductal adenocarcinoma cells. In some aspects, the cancerous cells are dissociated from human tissue. In some aspects, the human tissue is obtained by biopsy. In some aspects, the cells are stem cells.

In some aspects, the extracellular matrix is a basement membrane preparation secreted by Engelbreth-Holm-Swarm (EHS) mouse sarcoma cells. In some aspects, the extracellular matrix comprises laminin, entactin, and collagen IV. In some aspects, the extracellular matrix is Matrigel. In some aspects, the extracellular matrix is synthetic.

Disclosed are organoids obtained by the disclosed methods. In some aspects, the organoid is formed in vitro. In some aspects, at least 75%, 80%, 85%, 90%, 95%, or 98% of the cells in the organoid are viable. In some aspects, the organoid is maintained in culture for between at least 1 week and at least 1 month. In some aspects, the organoid is maintained in culture for over 1 month, 2 months, 3 months, 4 months, 5 months, or 6 months. In some aspects, the organoid is maintained in culture for at least 6 months, at least 7 months, at least 8 months, at least 9 months, at least 10 months, at least 11 months, or at least 12 months. In some aspects, the organoid is a pancreatic ductal adenocarcinoma organoid. In some aspects, the organoid is an organ fibrosis organoid. In some aspects, the organoid is a kidney fibrosis organoid. In some aspects, the organoid comprises a population of between at least 1×10³ cells and 1×10⁷ cells.

Disclosed are methods of conducting a drug discovery screen comprising culturing an organoid of any one of the present embodiments with a test compound. In some aspects, the assay comprises contacting the organoid with a test compound. The test compound may be a chemopreventive agent, cancer chemotherapeutic agent, environmental chemical, food supplement, or potential toxicant.

In one embodiment, provided herein are methods of conducting a toxicity assay comprising culturing an organoid of any one of the present embodiments. In some aspects, the assay determines the organ-specific cytotoxicity of test compounds. In some aspects, the assay comprises contacting the organoid with a test compound. The test compound may be a chemopreventive agent, cancer chemotherapeutic agent, environmental chemical, food supplement, or potential toxicant.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present disclosure will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

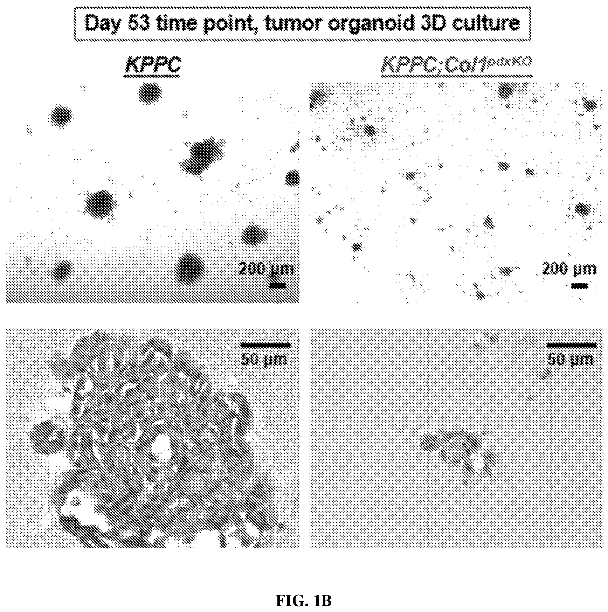

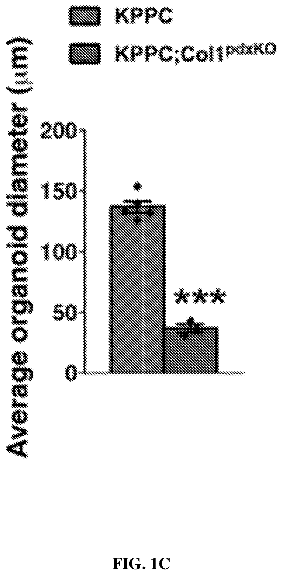

**FIGS. 1A-C** - FIG. 1A. Genetic strategy to delete type I collagen α1 (Col1a1) in cancer cell lineage in the context of pancreatic cancer using the *LSL-Kras^{G12D};Trp53*^{*loxP*/}*^{loxP};Pdx1-Cre;Col1a1*^{*loxP*/*loxP*} (referred to as KPPC;Col1^{pdxKO}) mice. The *LSL-Kras^{G12D};Trp53*^{*loxP*/}*^{loxP};Pdx1-Cre* (KPPC) mice were used as control animals. FIG. 1B. Organoids were established from tumor mixtures of KPPC and KPPC;Col1^{pdxKO} mice at the same age of 53 days. FIG. 1C. Average diameter of organoids from KPPC and KPPC;Col1^{pdxKO} tumors was quantified.

### DETAILED DESCRIPTION

Pieces of human tumors have been placed on plastic or placed on Matrigel to grow as organoids to study the biology of cancer, gene regulation or therapy efficacy/resistance, and drug screening. Organoids require collagen I homotrimers for their growth and progression to lesions resembling human cancer tissue. Cancer cells lacking collagen I homotrimers cannot form successful organoids. As such, collagen I homotrimers are necessary for the generation of organoids in culture, and supplementing cultures with collagen I homotrimers favors organoid formation.

### I. Organoid Generation and Culture

### A. Extracellular Matrices

Isolated stem cells or primary cells are preferably cultured in a microenvironment that mimics at least in part a cellular niche in which said stem cells or primary cells naturally reside. This cellular niche may be mimicked by culturing said cells in the presence of biomaterials, such as matrices, scaffolds, and culture substrates that represent key regulatory signals controlling cell fate. Such biomaterials comprise natural, semi-synthetic and synthetic biomaterials, and/or mixtures thereof. A scaffold provides a two-dimensional or three-dimensional network. Suitable synthetic materials for such a scaffold comprise polymers selected from porous solids, nanofibers, and hydrogels such as, for example, peptides including self-assembling peptides, hydrogels composed of polyethylene glycol phosphate, polyethylene glycol fumarate, polyacrylamide, polyhydroxyethyl methacrylate, polycellulose acetate, and/or co-polymers thereof. As is known to a skilled person, the mechanical properties such as, for example, the elasticity of the scaffold influences proliferation, differentiation, and migration of cells. Said scaffold is supplemented with natural, semi-synthetic or synthetic ligands, which provide the signals that are required for proliferation and/or differentiation, and/or migration of cells.

A cellular niche is in part determined by the stem cells or primary cells and surrounding cells, and the extracellular matrix (ECM) that is produced by the cells in said niche. In a preferred method of the invention, isolated tissue fragments or cells are attached to an ECM. ECM is composed of a variety of polysaccharides, water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated tissue fragments or cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C.

Alternatively, said ECM is commercially provided. Examples of commercially available extracellular matrices are extracellular matrix proteins (Invitrogen) and basement membrane preparations from Engelbreth-Holm-Swarm (EHS) mouse sarcoma cells (e.g. Matrigel^{™} (BD Biosciences)). A synthetic extracellular matrix material, such as ProNectin (Sigma Z378666) may be used. Mixtures of extracellular matrix materials may be used, if desired. The use of an ECM for culturing cells enhanced long-term survival of the cells. In the absence of an ECM, cell cultures could not be cultured for longer periods. In addition, the presence of an ECM allowed culturing of three-dimensional tissue organoids, which could not be cultured in the absence of an ECM. The extracellular matrix material will normally be coated onto a cell culture vessel, but may (in addition or alternatively) be supplied in solution.

A preferred ECM for use in a method of the invention comprises at least two distinct glycoproteins, such as two different types of collagen or a collagen and laminin. The ECM can be a synthetic hydrogel extracellular matrix or a naturally occurring ECM. A further preferred ECM is provided by Matrigel^{™} (BD Biosciences), which comprises laminin, entactin, and collagen IV.

The compositions of the disclosure may comprise serum or may be serum-free and/or serum-replacement free, as described elsewhere herein. Culture media and cell preparations are preferably GMP processes in line with standards required by the FDA for biologics products and to ensure product consistency.

### B. Culture Media

A cell culture medium that is used in a method of the invention comprises any suitable cell culture medium, subject to the limitations provided herein. Cell culture media typically contain a large number of ingredients, which are necessary to support maintenance of the cultured cells. Suitable combinations of ingredients can readily be formulated by the skilled person, taking into account the following disclosure. A culture medium according to the invention will generally be a nutrient solution comprising standard cell culture ingredients, such as amino acids, vitamins, inorganic salts, a carbon energy source, and a buffer, as described in more detail in the literature and below.

A culture medium will normally be formulated in deionized, distilled water. A culture medium will typically be sterilized prior to use to prevent contamination, e.g. by ultraviolet light, heating, irradiation, or filtration. The culture medium may be frozen (e.g. at - 20°C or -80°C) for storage or transport. The medium may contain one or more antibiotics to prevent contamination. The medium may have an endotoxin content of less than 0.1 endotoxin units per mL, or may have an endotoxin content less than 0.05 endotoxin units per mL. Methods for determining the endotoxin content of culture media are known in the art.

A preferred cell culture medium is a defined synthetic medium that is buffered at a pH of 7.4 (preferably with a pH 7.2-7.6 or at least 7.2 and not higher than 7.6) with a carbonate-based buffer, while the cells are cultured in an atmosphere comprising between 5% and 10% CO₂, or at least 5% and not more than 10% CO₂, preferably 5% CO₂.

The skilled person will understand from common general knowledge the types of culture media that might be used for as the basal medium in the cell culture mediums. Potentially suitable cell culture media are available commercially, and include, but are not limited to, Dulbecco's Modified Eagle Media (DMEM), Minimal Essential Medium (MEM), Knockout-DMEM (KO-DMEM), Glasgow Minimal Essential Medium (G-MEM), Basal Medium Eagle (BME), DMEM/Ham's F12, Advanced DMEM/Ham's F12, Iscove's Modified Dulbecco's Media and Minimal Essential Media (MEM), Ham's F-10, Ham's F-12, Medium 199, and RPMI 1640 Media. The basal medium may comprise Advanced DMEM F12, HEPES, penicillin/streptomycin, Glutamin, NAcetyl Cystein, B27, N2 and Gastrin.

It is furthermore preferred that said cell culture medium is supplemented with a purified, natural, semi-synthetic and/or synthetic growth factor and does not comprise an undefined component such as fetal bovine serum or fetal calf serum. Various different serum replacement formulations are commercially available and are known to the skilled person. Where a serum replacement is used, it may be used at between about 1% and about 30% by volume of the medium, according to conventional techniques.

In one embodiment, the tissue sample, for example, the pancreatic tissue sample, is incubated in a cell culture medium. In one embodiment, the cell culture medium is Dulbecco's Modified Eagle Medium (DMEM). In another embodiment, the cell culture medium is Dulbecco's Modified Eagle Medium/Nutrient Mixture F-12 (DMEM/F-12). In another embodiment, the cell culture medium is supplemented with serum. In one embodiment, the cell culture medium is supplemented with fetal bovine serum (FBS).

In one embodiment, the tissue sample, for example, the pancreatic tissue sample, is dissociated enzymatically. In one embodiment, the tissue sample is dissociated enzymatically by incubation of tissue with cell culture medium supplemented with collagenase. Collagenase can break down the collagen found in tissues. In one embodiment, the final concentration of collagenase in the cell culture medium is 300 units/ml. In another embodiment, the final concentration of collagenase in the ceil culture medium is at least 50 units/ml, at least 100 units/ml, at least 200 units/ml, at least 300 units/ml, at least 400 units/ml, at least 500 units/ml, at least 600 units/ml, at least 700 units/ml, at least 800 units/ml, at least 900 units/ml, or at least 1000 units/ml.

In one embodiment, dissociated tissue, for example, dissociated pancreatic tissue, is separated from the dissociating medium by centrifugation. In one embodiment, the tissue can be further dissociated by incubation of the tissue with trypsin. Trypsin is a serine protease and can hydrolyze proteins. In one embodiment, the trypsin is added to prostate tissue at a final concentration of 6.25 mg/1. In another embodiment, the final concentration of trypsin is at least l mg/1, at least 2 mg/l, at least 3 mg 1, at least 4 mg/l, at least 5 mg/l, at least 6 mg/l, at least 7 mg/l, at least 8 mg/l, at least 9 mg/l, at least 10 mg/l, at least 11 mg/1, at least 12 mg/1, at least 13 mg/'l, at least 14 mg/l, at least 15 mg/l, at least 16 mg/l, at least 17 mg/l, at least 18 mg/l, at least 19 mg/l, or at least 20 mg/l In one embodiment, trypsin is added in Hank's Balanced Salt Solution (HBSS) and EDTA. In one embodiment, the sample is incubated on ice for 1 hour. In one embodiment, the sample is incubated for at least 5 minutes, at least 10 minutes, at least 15 minutes, at least 20 minutes, at least 30 minutes, at least 45 minutes, at least 1 hour, at least 2 hours, at least 3 hours, at least 4 hours, or at least 5 hours.

The media described above can be supplemented as necessary with supplementary components or ingredients, including optional components, in appropriate concentrations or amounts, as necessary or desired. Cell medium solutions provide at least one component from one or more of the following categories: (1) an energy source, usually in the form of a carbohydrate such as glucose; (2) all essential amino acids, and usually the basic set of twenty amino acids plus cysteine; (3) vitamins and/or other organic compounds required at low concentrations; (4) free fatty acids or lipids; and (5) trace elements, where trace elements are defined as inorganic compounds or naturally occurring elements that are typically required at very low concentrations, usually in the micromolar range.

The medium also can be supplemented electively with one or more components from any of the following categories: (1) salts, for example, magnesium, calcium, and phosphate; (2) hormones and other growth factors such as, serum, insulin, transferrin, epidermal growth factor and fibroblast growth factor; (3) protein and tissue hydrolysates, for example peptone or peptone mixtures which can be obtained from purified gelatin, plant material, or animal byproducts; (4) nucleosides and bases such as, adenosine, thymidine, and hypoxanthine; (5) buffers, such as HEPES; (6) antibiotics, such as gentamycin or ampicillin; (7) cell protective agents; and (8) galactose.

Cells maintained in culture can be passaged by their transfer from a previous culture to a culture with fresh medium. In one embodiment, induced cells are stably maintained in cell culture for at least 3 passages, at least 4 passages, at least 5 passages, at least 6 passages, at least 7 passages, at least 8 passages, at least 9 passages, at least 10 passages, at least 11 passages, at least 12 passages, at least 13 passages, at least 14 passages, at least 15 passages, at least 20 passages, at least 25 passages, or at least 30 passages.

In one embodiment, the culture medium is supplemented with 5% Matrigel^{™}. In one embodiment, the culture medium is supplemented with about 0.1% Matrigel^{™}, about 0.2% Matrigel^{™}, about 0.3% Matrigel^{™}, about 0.4% Matrigel^{™}, about 0.5% Matrigel^{™}, about 0.6% Matrigel^{™}, about 0.7% Matrigel^{™}, about 0.8% Matrigel^{™}, about 0.9% Matrigel^{™}, about 1% Matrigel^{™}, about 2% Matrigel^{™}, about 3% Matrigel^{™}, about 4% Matrigel^{™}, about 5% Matrigel^{™}, about 6% Matrigel^{™}, about 7% Matrigel^{™}, about 8% Matrigel^{™}, about 9% Matrigel^{™}, about 10% Matrigel^{™}, about 15% Matrigel^{™}, or about 20% Matrigel^{™}. In one embodiment, the culture medium is supplemented with at least 0.1% Matrigel^{™}, at least 0.2% Matrigel^{™}, at least 0.3% Matrigel^{™}, at least 0.4% Matrigel^{™}, at least 0.5% Matrigel^{™}, at least 0.6% Matrigel^{™}, at least 0.7% Matrigel^{™}, at least 0.8% Matrigel^{™}, at least 0.9% Matrigel^{™}, at least 1% Matrigel^{™}, at least 2% Matrigel^{™}, at least 3% Matrigel^{™}, at least 4% Matrigel^{™}, at least 5% Matrigel^{™}, at least 6% Matrigel^{™}, at least 7% Matrigel^{™}, at least 8% Matrigel^{™}, at least 9% Matrigel^{™}, at least 10% Matrigel^{™}, or at least 20% Matrigel^{™}.

In one embodiment, the culture medium is supplemented with 5% FBS. In another embodiment, the FBS is heat-inactivated charcoal- stripped FBS (e.g. Gibco, cat #12676). In one embodiment the culture medium is supplemented with about 0.1% FBS, about 0.2% FBS, about 0.3% FBS, about 0.4% FBS, about 0.5% FBS, about 0.6% FBS, about 0.7% FBS, about 0.8% FBS, about 0.9% FBS, about 1% FBS, about 2% FBS, about 3% FBS, about 4% FBS, about 5% FBS, about 6% FBS, about 7% FBS, about 8% FBS, about 9% FBS, about 10% FBS, about 15% FBS, or about 20% FBS, or more. In one embodiment, the culture medium is supplemented with at least 0.1% FBS, at least 0.2% FBS, at least 0.3% FBS, at least 0.4% FBS, at least 0.5% FBS, at least 0.6% FBS, at least 0.7% FBS, at least 0.8% FBS, at least 0.9% FBS, at least 1% FBS, at least 2% FBS, at least 3% FBS, at least 4% FBS, at least 5% FBS, at least 6% FBS, at least 7% FBS, at least 8% FBS, at least 9% FBS, at least 10% FBS, or at least 20% FBS.

In one embodiment, cells can be cultured to generate organoids using a Matrigel^{™} floating method. In another embodiment, cells can be cultured to generate organoids using a Matrigel^{™} embedding method. In one embodiment, organoids can be grown for at least 3 weeks. In further embodiments, organoids can be growth for at least 1 week, at least 2 weeks, at least 3 weeks, at least 4 weeks, at least 5 weeks, at least 6 weeks, at least 7 weeks, at least 8 weeks, at least 3 months, at least 4 months, at least 5 months, at least 6 months, at least 7 months, or at least 8 months. The invention also provides an organoid which has been cultured for at least 6, 8, 10, 12, 14, 16, 18, or 20 weeks.

### II. Organoids and Uses Thereof

In one embodiment, the organoid is a pancreas organoid. In one embodiment, the organoid is a prostate organoid. In another embodiment, the organoid is a bladder organoid. In another embodiment, the organoid is a liver organoid. In yet another embodiment, the organoid is a kidney organoid. In a further embodiment, the organoid is a breast organoid. In another embodiment, the organoid is a lung organoid. In one embodiment, the organoid is a heart organoid. In another embodiment, the organoid is a skin organoid. In one embodiment, the organoid is a stomach organoid. In another embodiment, the organoid is a brain organoid. In another embodiment, the organoid is a colon organoid. In yet another embodiment, the organoid is an intestine organoid. In yet other embodiments, any organoid can be used in the methods of the invention.

The present disclosure provides methods for dissociating cells from a tissue or mixed population of cells. In one embodiment, cells are dissociated from pancreatic tissue. In one embodiment, cells are dissociated from prostate tissue. In another embodiment, cells are dissociated from bladder tissue. In another embodiment, cells are dissociated from liver tissue. In yet another embodiment, cells are dissociated from kidney tissue. In a further embodiment, cells are dissociated from breast tissue. In another embodiment, cells are dissociated from lung tissue. In one embodiment, cells are dissociated from heart tissue. In another embodiment, cells are dissociated from skin tissue. In one embodiment, cells are dissociated from stomach tissue. In another embodiment, cells are dissociated from brain tissue. In another embodiment, cells are dissociated from colon tissue. In another embodiment, cells are dissociated from intestinal tissue.

In one embodiment, cells are dissociated from normal tissue. In one embodiment, cells are dissociated from non-cancerous tissue. In another embodiment, cells are dissociated from cancerous tissue. In another embodiment, cells are dissociated from human tissue. In a further embodiment, cells are dissociated from mouse tissue. In other embodiments, cells are dissociated from tissue from any mammal. In one embodiment, cells are dissociated from localized tumors. In another embodiment, cells are dissociated from malignant tumors. In another embodiment, cells are dissociated from metastasized tumors.

In a further embodiment, the organoids are cultured from one or more localized tumors. In one embodiment, the organoids are cultured from malignant tumors. In another embodiment, the organoids are cultured from metastasized tumors. In one embodiment, the tumor is a prostate tumor. In another embodiment, the tumor is a pancreas, breast, heart, lung, liver, bladder, kidney, skin, stomach, brain, colon, or intestinal tumor.

In one embodiment, a sample of tissue can be obtained by biopsy. Methods of obtaining tissue samples are known to one of skill in the art. In one embodiment, the sample of tissue is obtained from a pancreas biopsy. In one embodiment, the sample of tissue is obtained from a bladder biopsy. In another embodiment, the sample of tissue is obtained from a liver biopsy. In yet another embodiment, the sample of tissue is obtained from a kidney biopsy. In a further embodiment, the sample of tissue is obtained from a breast biopsy. In another embodiment, the sample of tissue is obtained from a lung biopsy. In one embodiment, the sample of tissue is obtained from a heart biopsy. In another embodiment, the sample of tissue is obtained from a skin biopsy. In one embodiment, the sample of tissue is obtained from a stomach biopsy. In another embodiment, the sample of tissue is obtained from a brain biopsy. In one embodiment, the sample of tissue is obtained from a colon biopsy. In another embodiment, the sample of tissue is obtained from an intestine biopsy. In some embodiments, a sample of tissue is obtained by fine needle aspiration biopsy. In another embodiment, a sample of tissue is obtained by core needle biopsy. In a further embodiment, a sample of tissue is obtained by surgical biopsy.

In one embodiment, the subject is an animal. In certain embodiments, the subject is a human. In other embodiments, the subject is a mammal. In some embodiments, the subject is a rodent, such as a mouse or a rat. In another embodiment, the subject is a mouse. In one embodiment, the mouse is a genetically-engineered mouse. In some embodiments, the subject is a cow, pig, sheep, goat, cat, horse, dog, and/or any other species of animal used as livestock or kept as pets.

In one embodiment, cell cultures that grow as attached cells in two-dimensional culture are derived from the organoids. In one embodiment, the cells are cancerous. In another embodiment, the cells are tumor cells. In another embodiment, the cells are normal. In yet another embodiment, the cells are non-cancerous.

In one embodiment, cells are dissociated from a tissue sample by cutting into small chunks. In one embodiment, the tissue sample is a pancreatic tissue sample. In another embodiment, the tissue sample is a breast, heart, lung, liver, bladder, kidney, breast, skin, stomach, brain, prostate, colon, or intestine tissue sample. In another embodiment 1 gram of tissue is used. In one embodiment, at least 0.1 gram, at least 0,2. grams, at least 0.3 grams, at least 0.4 grams, at least 0.5 grams, at least 0.6 grams, at least 0.7 grams, at least 0.8 grams, at least 0.9 grams, at least 1.0 grams, at least 2.0 grams, at least 3.0 grams, at least 4,0 grams, at least 5.0 grams, at least 6.0 grams, at least 7.0 grams, at least 8.0 grams, at least 9.0 grams, or at least 10.0 grams of tissue is used. In another embodiment, the whole organ is used.

An organoid according to the present invention may comprise a population of cells of at least 1×10³ cells, at least 1×10⁴ cells, at least 1×10⁵ cells, at least 1×10⁶ cells, at least 1×10⁷ cells or more. In some embodiments, each organoid comprises between approximately 1×10³ cells and 5×10³ cells; generally, 10-20 organoids may be grown together in one well of a 24 well plate.

An organoid may be an organoid that is still being cultured using a method of the invention and is therefore in contact with an extracellular matrix. Preferably, an organoid is embedded in an extracellular matrix. Within the context of the invention, "in contact" means a physical or mechanical or chemical contact, which means that for separating said organoid from said extracellular matrix a force needs to be used.

An organoid may be cultured during at least 2, 3, 4, 5, 6, 7, 8, 9, 10 weeks or 1, 2, 3, 4, 5, 6, 7, 8, 9, 10, 11, or 12 months or longer.

The invention further provides an organoid, preferably comprising at least 50% viable cells, more preferred at least 60% viable cells, more preferred at least 70% viable cells, more preferred at least 80% viable cells, more preferred at least 90% viable cells. Viability of cells may be assessed using Hoechst staining or Propidium Iodide staining in FACS.

Described is the use of a cell or organoid as described above in a drug discovery screen, toxicity assay or in regenerative medicine. Further described is the use of the progeny of organoids, in toxicity assays. Such toxicity assays may be in vitro assays using a cell derived from an organoid or an organoid or part thereof. Such progeny and organoids are easy to culture and more closely resemble primary cells than, for example, cell lines that are currently used in toxicity assays. It is anticipated that toxicity results obtained with organoids more closely resemble results obtained in patients. A cell-based toxicity test is used for determining organ specific cytotoxicity. Compounds that are tested in said test comprise cancer chemopreventive agents, cancer chemotherapeutic agents, environmental chemicals, food supplements, and potential toxicants. The cells are exposed to multiple concentrations of a test agent for certain period of time. The concentration ranges for test agents in the assay are determined in a preliminary assay using an exposure of five days and log dilutions from the highest soluble concentration. At the end of the exposure period, the cultures are evaluated for inhibition of growth. Data are analyzed to determine the concentration that inhibited end point by 50 percent (TC₅₀).

For example, according to this aspect, a candidate compound may be contacted with a cell or an organoid as described herein, and any change to the cells or in to activity of the cells may be monitored. Examples of other non-therapeutic uses of the cells or organoids of the present invention include research of embryology, cell lineages, and differentiation pathways; gene expression studies including recombinant gene expression; mechanisms involved in tissue injury and repair; research of inflammatory and infectious diseases; studies of pathogenetic mechanisms; and studies of mechanisms of cell transformation and etiology of cancer.

For high-throughput purposes, the organoids are cultured in multiwell plates such as, for example, 96 well plates or 384 well plates. Libraries of molecules are used to identify a molecule that affects the organoids. Preferred libraries comprise antibody fragment libraries, peptide phage display libraries, peptide libraries (e.g. LOPAP^{™}, Sigma Aldrich), lipid libraries (BioMol), synthetic compound libraries (e.g. LOP AC^{™}, Sigma Aldrich), or natural compound libraries (Specs, TimTec). Furthermore, genetic libraries can be used that induce or repress the expression of one of more genes in the progeny of the cells. These genetic libraries comprise cDNA libraries, antisense libraries, and siRNA or other non-coding RNA libraries. The cells are preferably exposed to multiple concentrations of a test agent for certain period of time. At the end of the exposure period, the cultures are evaluated. The term "affecting" is used to cover any change in a cell, including, but not limited to, a reduction in, or loss of, proliferation, a morphological change, and cell death. The organoids can also be used to identify drugs that specifically target carcinoma cells, but not the organoids.

Described is a method for identifying a compound that inhibits cancer, the method comprising: (a) contacting an organoid with a test compound; (b) determining whether growth of the organoid is inhibited in the presence of the test compound, as compared to growth of the organoid in the absence of the test compound; wherein inhibition of growth of the organoid indicates the identification of a compound that inhibits cancer.

Furthermore, the organoids can be used for culturing of a pathogen.

Described are tumor tissue banks in which patient-specific organoids can be stored and used for the large-scale screening of candidate therapeutic compounds. Such organoid banks can also be useful for patient-specific diagnostics, assays for the efficacy of potential treatments, and identification of the appropriate targeted tumor population (cancer stem cells), as well as other applications in personalized medicine.

In the present disclosure, reference to "cells" is context dependent and may include primary cells, including cells obtained by tissue biopsy and other known methods of obtaining cells from a subject and cells from cell lines and other similar sources of cultured cells.

### III. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Collagen 1 Homotrimers Support Organoid Formation

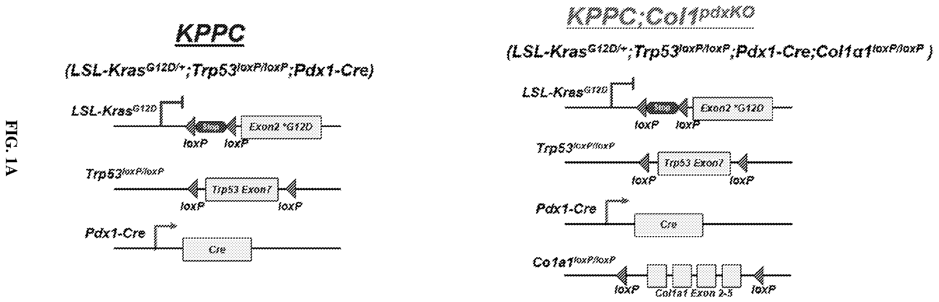

The *LSL-Kras^{G12D};Pdx1-Cre* (referred to as KC) or *LSL-Kras^{G12D};Trp53*^{*loxP*/}*^{loxP};Pdx1-Cre* (referred to as KPPC) mice were crossed with the *Col1a1*^{*loxP*/*loxP*} mouse strain (with loxP-flanked exons 2-5; established from the Col1a1^{tm1a(EUCOMM)Wtsi} strain that was purchased from European Mouse Mutant Cell Repository (EuMMCR)), resulting in the generation of the KC;Col1a1^{loxP/loxP} (referred to as KC;Col1^{pdxKO}) and KPPC;Col1a1^{loxP/loxP} (referred to as KPPC;Col1^{pdxKO}) mice (FIG. 1A). These mice allow the Col1a1 deletion in PDAC cells. The aforementioned experimental mice with desired genotypes were monitored and analyzed with no randomization or blinding. Both female and male mice with desired genotype(s) for PDAC were used for experimental mice. All mice were housed under standard housing conditions at MD Anderson Cancer Center (MDACC) animal facilities, and all animal procedures were reviewed and approved by the MDACC Institutional Animal Care and Use Committee.

Age-matching tumors (at the same age of 53 days) from KPPC and KPPC;Col1F/F mice were collected for 3D organoid culture on matrigel. After one week culture, the KPPC;Col1F/F organoid was growing significantly smaller than that of KPPC control mice, as expected.

PDAC organoids in 3D matrigel were established from tumor tissues of KPPC;Col1^{pdxKO} mouse and KPPC control mouse, respectively. KPPC;Col1^{pdxKO} organoids revealed significantly impeded growth as compared with KPPC organoids (FIGS. 1B&C). In addition, several different methods were tested in parallel for making organoids on gel into paraffin-embedded samples, which is good for long-term preservation and serial sections. A manual dehydrating-embedding method worked. Sections were obtained from these paraffin-embedded organoid samples and stained for H&E. Meanwhile, primary cancer cell lines (KPPC and KPPC;Col1F/F ) were also generated from these mice using 2D dishes. These cell lines will also be tested for organoid formation.

All of the methods disclosed and claimed herein can be made and executed without undue experimentation in light of the present disclosure.

### REFERENCES

Boj et al., "Organoid models of human and mouse ductal pancreatic cancer," Cell, 160:324-338, 2015.

## Claims

1. A method for obtaining and/or culturing an organoid, the method comprising culturing pancreatic ductal adenocarcinoma cells with an extracellular matrix in the presence of a medium, additive, or surface comprising exogenous collagen I homotrimers.

2. The method of claim 1, wherein the cancerous cells are dissociated from human tissue.

3. The method of claim 2, wherein the human tissue is obtained by biopsy.

4. The method of claim 1, wherein the cells are stem cells.

5. The method of claim 1, wherein the extracellular matrix is a basement membrane preparation secreted by Engelbreth-Holm-Swarm (EHS) mouse sarcoma cells.

6. The method of claim 5, wherein the extracellular matrix comprises laminin, entactin, and collagen IV.

7. The method of claim 1, wherein the method comprises culturing cells with an extracellular matrix in the presence of a basal cell culture medium for animal or human cells, the medium comprising exogenous collagen I homotrimers.

8. The method of claim 7, wherein the medium is serum free, the medium comprises a serum replacement, or the medium is supplemented with serum.

9. The method of claim 1, wherein the method comprises culturing cells with an extracellular matrix in the presence of a cell culture additive for animal or human cells, the additive comprising exogenous collagen I homotrimers.

10. The method of claim 9, wherein the additive is an extracellular matrix, preferably Matrigel, a hydrogel, a biological scaffold, or a synthetic scaffold.

11. The method of claim 1, wherein the method comprises culturing cells with an extracellular matrix in the presence of a cell culture surface for animal or human cells, the surface comprising exogenous collagen I homotrimers.

12. The method of claim 11, wherein the surface is a cell culture plate, a channel, a duct, a valve, or a microfluidic device.

## Patentansprüche

1. Ein Verfahren zur Gewinnung und/oder Kultivierung eines Organoids, wobei das Verfahren die Kultivierung von Pankreas-Duktaladenokarzinomzellen mit einer extrazellulären Matrix in Gegenwart eines Mediums, Additivs oder einer Oberfläche umfasst, die exogene Kollagen-I-Homotrimere umfasst.

2. Das Verfahren nach Anspruch 1, wobei die Krebszellen aus menschlichem Gewebe dissoziiert werden.

3. Das Verfahren nach Anspruch 2, wobei das menschliche Gewebe durch Biopsie gewonnen wird.

4. Das Verfahren nach Anspruch 1, wobei die Zellen Stammzellen sind.

5. Das Verfahren nach Anspruch 1, wobei die extrazelluläre Matrix eine Basalmembranpräparation ist, die von Engelbreth-Holm-Swarm (EHS)-Maussarkomzellen sezerniert wird.

6. Das Verfahren nach Anspruch 5, wobei die extrazelluläre Matrix Laminin, Entactin und Kollagen IV umfasst.

7. Das Verfahren nach Anspruch 1, wobei das Verfahren die Kultivierung von Zellen mit einer extrazellulären Matrix in Gegenwart eines basalen Zellkulturmediums für tierische oder menschliche Zellen umfasst, wobei das Medium exogene Kollagen-I-Homotrimere umfasst.

8. Das Verfahren nach Anspruch 7, wobei das Medium serumfrei ist, das Medium einen Serumersatz umfasst oder das Medium mit Serum angereichert wird.

9. Das Verfahren nach Anspruch 1, wobei das Verfahren die Kultivierung von Zellen mit einer extrazellulären Matrix in Gegenwart eines Zellkulturadditivs für tierische oder menschliche Zellen umfasst, wobei das Additiv exogene Kollagen-I-Homotrimere umfasst.

10. Das Verfahren nach Anspruch 9, wobei das Additiv eine extrazelluläre Matrix, vorzugsweise Matrigel, ein Hydrogel, ein biologisches Gerüst oder ein synthetisches Gerüst ist.

11. Das Verfahren nach Anspruch 1, wobei das Verfahren die Kultivierung von Zellen mit einer extrazellulären Matrix in Gegenwart einer Zellkulturoberfläche für tierische oder menschliche Zellen umfasst, wobei die Oberfläche exogene Kollagen-I-Homotrimere umfasst.

12. Das Verfahren nach Anspruch 11, wobei die Oberfläche eine Zellkulturplatte, ein Kanal, eine Röhre, ein Ventil oder eine mikrofluidische Vorrichtung ist.

## Revendications

1. Procédé d'obtention et/ou de culture d'un organoïde, le procédé comprenant
la culture de cellules d'adénocarcinome canalaire pancréatique avec une matrice extracellulaire en présence
d'un milieu, d'un additif ou d'une surface comprenant des homotrimères de collagène I exogènes.

2. Procédé selon la revendication 1, dans lequel les cellules cancéreuses sont dissociées d'un tissu humain.

3. Procédé selon la revendication 2, dans lequel le tissu humain est obtenu par biopsie.

4. Procédé selon la revendication 1, dans lequel les cellules sont des cellules souches.

5. Procédé selon la revendication 1, dans lequel la matrice extracellulaire est une préparation de membrane basale sécrétée par des cellules de sarcome de souris Engelbreth-Holm-Swarm (EHS).

6. Procédé selon la revendication 5, dans lequel la matrice extracellulaire comprend de la laminine, de l'entactine et du collagène IV.

7. Procédé selon la revendication 1, dans lequel le procédé comprend la culture de cellules avec une matrice extracellulaire en présence d'un milieu de culture cellulaire basal pour cellules animales ou humaines, le milieu comprenant des homotrimères de collagène I exogènes.

8. Procédé selon la revendication 7, dans lequel le milieu est sans sérum, le milieu comprend un substitut de sérum, ou le milieu est supplémenté avec du sérum.

9. Procédé selon la revendication 1, dans lequel le procédé comprend la culture de cellules avec une matrice extracellulaire en présence d'un additif de culture cellulaire pour cellules animales ou humaines, l'additif comprenant des homotrimères de collagène I exogènes.

10. Procédé selon la revendication 9, dans lequel l'additif est une matrice extracellulaire, de préférence du Matrigel, un hydrogel, un support biologique ou un support synthétique.

11. Procédé selon la revendication 1, dans lequel le procédé comprend la culture de cellules avec une matrice extracellulaire en présence d'une surface de culture cellulaire pour cellules animales ou humaines, la surface comprenant des homotrimères de collagène I exogènes.

12. Procédé selon la revendication 11, dans lequel la surface est une plaque de culture cellulaire, un canal, un conduit, une valve ou un dispositif microfluidique.
